# EUROPÄISCHE PATENTSCHRIFT

(11) **EP 0 699 652 B1**
(45) Veröffentlichungstag und Bekanntmachung des Hinweises auf die Patenterteilung: **11.11.1998**
(21) Anmeldenummer: 95113099.6
(22) Anmeldetag: 21.08.1995
(51) Int. Cl.: C07C 29/17, C07C 31/20, C07C 67/465, C07C 69/60

(54) **Verfahren zur Herstellung von Butandiol-1,4 aus Maleinsäureanhydrid**
Method for the preparation of butane-1,4-diol from maleic anhydride
Procédé pour la préparation de butane-1,4-diol à partir de l'anhydride maléique

(30) Priorität: 02.09.1994 DE 4431220
(43) Veröffentlichungstag der Anmeldung: 06.03.1996
(73) Patentinhaber: BAYER AG, 51368 Leverkusen (DE)
(72) Erfinder: Darsow, Gerhard, Dr., D-47804 Krefeld (DE)

(56) Entgegenhaltungen:
- EP-A- 0 447 963
- WO-A-82/03854
- WO-A-86/03189
- WO-A-87/07280
- FR-A- 2 371 406
- GB-A- 1 454 440
- US-A- 5 196 602

## Beschreibung

Die vorliegende Erfindung betrifft ein Verfahren zur Herstellung von Butandiol-1,4 aus Maleinsäureanhydrid (MSA), in welchem MSA zunächst mit Butandiol-1,4 oligoverestert wird und der entstandene Oligoester in zwei Hydrierschritten an zwei unterschiedlichen Katalysatoren und in zwei unterschiedlichen Temperaturbereichen bei erhöhtem Druck und in Gegenwart von überschüssigem Wasserstoff zu Butandiol-1,4 hydriert wird.

Butandiol-1,4 ist ein wichtiges Monomer für die Produktion von thermoplastischen Polyestern, beispielsweise von Polybutylenterephthalat PBT, sowie von Polyurethanen mit besonderen mechanischen und chemischen Eigenschaften, außerdem Zwischenprodukt für Tetrahydrofuran und elastische Faserstoffe, die unter Verwendung von Polytetramethylenetherglykol PTMEG hergestellt werden.

Es ist bereits bekannt, MSA direkt oder in Form der Maleinsäure in wäßriger Lösung zu hydrieren, wobei gemäß Angaben in GB 1.587.198 neben Butandiol-1,4 stets größere Mengen an Tetrahydrofuran und/oder γ-Butyrolacton entstehen. Es ist weiterhin bekannt, MSA mit Monoalkoholen zunächst zu einem Dialkyl-maleinat zu verestern und dieses in einer einstufigen Reaktionszone zu einem Gemisch von Butandiol-1,4 und Tetrahydrofuran zu hydrieren, wobei sowohl in der Flüssigphase (GB 1.454.440) als auch in der Gasphase gearbeitet werden kann (WO 82/03854), wobei γ-Butyrolacton als Zwischenprodukt beobachtet wird. Als Katalysatoren werden Kupferchromit bzw. Cu/Zn-Katalysatoren eingesetzt. Die Hydrierung des Dialkyl-maleinats ist auch bereits zweistufig beschrieben worden (WO 86/03189), wobei in beiden Stufen in der Gasphase an Kupferchromit-Katalysatoren gearbeitet wird.

In den bisher bekannt gewordenen Verfahren treten stets so große Mengen an Nebenprodukten auf, daß diese einen erheblichen Aufwand bei der Reindarstellung des Reaktionsproduktes verursachen. Eine Recyclisierung ist nicht immer möglich, da die Hydrierung zum gewünschten Endprodukt nicht immer glatt verläuft und daher zu einem höheren Niveau an im Kreis geführten Nebenprodukten führt. Eine grundsätzliche Schwierigkeit bereitet ferner der zur Veresterung eingesetzte Monoalkohol, der grundsätzlich vom Endprodukt abgetrennt werden muß. Das Festhalten an Monoalkoholen zur Veresterung erklärt sich ganz offenbar aus der Tatsache, daß bei der Fortentwicklung des Hydrierverfahrens von der flüssigen zur gasförmigen Phase ein leicht verdampfbarer Ester zur Verfügung stehen mußte. Für einen Diester der Maleinsäure aus Monoalkoholen spricht ferner die Tatsache, daß solche Ester in übersichtlicher Weise rein dargestellt werden können, da bei Hydrierungen verunreinigte Ausgangsmaterialien in der Regel zu Schwierigkeiten führen.

Es wurde nun gefunden, daß es gegen diese vorherrschende Meinung möglich ist, unter Verwendung des systemeigenen Butandiols-1,4 einen Oligoester herzustellen, der bekanntermaßen keinen einheitlichen Stoff darstellt, und diesen, bevorzugt ohne weitere Vorreinigung, der Hydrierung zu Butandiol-1,4 zuzuführen, wobei diese Hydrierung in zwei Hydrierschritten an verschiedenen Hydrierkatalysatoren und in verschiedenen Bereichen der Hydriertemperatur durchgeführt wird. Dieses Verfahren hat sich als sehr kostengünstig erwiesen. Es ist ferner ökologisch vorteilhaft, da kein systemfremder Stoff, wie ein Monoalkohol, benutzt wird und weil nur sehr geringe Mengen der üblicherweise auftretenden, entsorgungsbedürftigen Nebenprodukte beobachtet werden.

Die Erfindung betrifft ein Verfahren zur Herstellung von Butandiol-1,4 aus Maleinsäureanhydrid (MSA) durch Veresterung des MSA und katalytische Hydrierung des entstandenen Esters in der Flüssigphase, das dadurch gekennzeichnet ist, daß man
a) zur Veresterung des MSA Butandiol-1,4 im molaren Verhältnis Butandiol-1,4:MSA = 1,1-2:1, bevorzugt 1,15-1,5:1 einsetzt,
b) die Veresterung unter Abdestillieren des herausgespaltenen Wassers diskontinuierlich oder kontinuierlich in 1-4 Veresterungsstufen im Temperaturbereich von 100-130°C, bevorzugt 105-125°C und im Druckbereich von 1500-100 mbar durchführt,
c) den im Schritt b) gebildeten Oligoester in einem ersten Hydrierschritt im Temperaturbereich von 60-130°C an einem trägerfreien Katalysator aus verpreßtem Metallpulver von Ni, Fe, Co oder Gemischen daraus mit überschüssigem Wasserstoff behandelt und
d) in einem zweiten Hydrierschritt im Temperaturbereich von 190-230°C an einem reduzierten, trägerfreien Katalysator aus verpreßten Pulvern von Cu-Oxiden, ZnO und Al₂O₃ mit Anteilen von Oxiden des Ni, Fe, Co oder Gemischen mehrerer von ihnen die Hydrierung vervollständigt, wobei der Temperaturunterschied zwischen den Schritten c) und d) 60-130°C beträgt, ein 20-100facher molarer H₂-Überschuß angewandt wird und die Schritte c) und d) bei gleichem oder unterschiedlichem Druck im Bereich von 100-400 bar, bevorzugt 150-300 bar, durchgeführt werden.

Der Reaktionsverlauf über alle Schritte läßt sich durch das folgende Formelschema veranschaulichen:

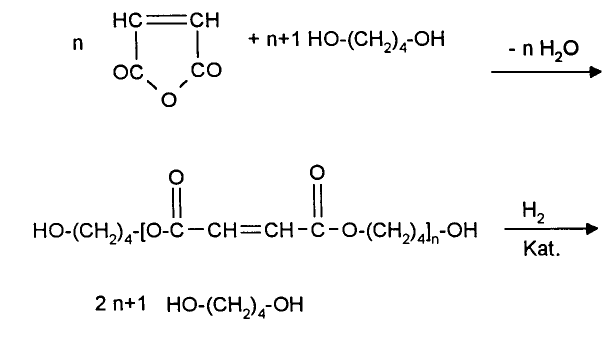

Verestert man Maleinsäureanhydrid mit einem Monoalkohol, wie es bereits bekannte Verfahren vorsehen, um niedrig siedende Dialkylmaleinate, wie Dimethylmaleinat, Diethylmaleinat, Dipropylmaleinat usw. zu erhalten, muß in der Regel in Gegenwart eines Veresterungskatalysators und eines Schleppmittels für das bei der Veresterung herausgespaltene Wasser, beispielsweise Toluol, gearbeitet werden. Damit enthält ein solches Verfahren drei systemfremde Stoffe, nämlich den Monoalkohol, den Veresterungskatalysator und das Schleppmittel, die allesamt nach Beendigung der Gesamtreaktion, vielfach schon vor dem Hydrierschritt abgetrennt und wieder aufgearbeitet werden müssen. Dies erfordert einen hohen Aufwand an Energie, weil beispielsweise das Schleppmittel ein Vielfaches der abgespaltenen Wassermenge beträgt. Auch die Reinigung des Dialkylmaleinats vor der katalytischen Hydrierung, beispielsweise durch Destillation oder andere umständliche Methoden, erfordert einen beträchtlichen Energieaufwand. Nach der hydrogenolytischen Spaltung eines solchen Dialkylmaleinats tritt der eingesetzte Monoalkohol wieder in Erscheinung und muß von den Reaktionsprodukten Butandiol-1,4, Tetrahydrofuran und γ-Butyrolacton getrennt und zur Wiederverwendung in der Veresterungsstufe gereinigt werden. Zusätzlich sind nicht zu vermeidende Verluste zu ergänzen. Für den Fall, daß ein Monoalkohol mit mehr als drei C-Atomen zur Veresterung eingesetzt werden soll, kann möglicherweise auf den Veresterungskatalysator und das Schleppmittel verzichtet werden. Solche höheren Monoalkohole sind jedoch teurer als die niedrigeren Monoalkohole, so daß nicht zu vermeidende Verluste die Kostenseite eines solchen Verfahrens stärker belasten. Wegen der höheren Siedepunkte ist zusätzlich jeder Destillationsaufwand größer. Die grundsätzliche Belastung eines solchen Verfahrens mit dem systemfremden Stoff Monoalkohol kann in keinem Falle vermieden werden.

Anders und vorteilhaft liegt der Fall, wenn zur Veresterung des MSA erfindungsgemäß Butandiol-1,4 eingesetzt wird, welches als systemeigener Stoff im Reaktionsprodukt verbleiben kann. Von diesem Reaktionsprodukt kann ein benötigter Teil Butandiol-1,4 abgezweigt und zur Veresterung von weiterem MSA eingesetzt werden. Das zur Veresterung benötigte Butandiol-1,4 kann jedoch grundsätzlich auch aus anderer Quelle, beispielsweise in verunreinigter Form, stammen.

Arbeitet man bei der Veresterung von MSA mit Butandiol-1,4 unter den in der Literatur bekannten Veresterungstemperaturen von 150-250°C, so bleibt die Veresterung bei einem stöchiometrischen Ansatz im Molverhältnis 1:1 jedoch unvollständig, weil neben der Veresterung in Abhängigkeit vom sich intermediär bildenden oder von vornherein vorhandenen Gehalt an Maleinsäure im Reaktionsgemisch als Nebenreaktion eine säurekatalysierte Bildung von Tetrahydrofuran aus dem eingesetzten Butandiol-1,4 eintritt. Ein vollständiger Umsatz des MSA bzw. der im Reaktionsgemisch enthaltenen Maleinsäure erscheint nur dann möglich, wenn erhebliche stöchiometrische Überschüsse an Butandiol-1,4 zum Einsatz kommen. Aber auch in einem solchen Fall entstehen noch beträchtliche Anteile an Tetrahydrofuran, beispielsweise bei der Veresterung von Butandiol-1,4 und MSA im Molverhältnis 3:1 bei 180°C noch 15,4 Gew.-% Tetrahydrofuran als Nebenprodukt aus dem eingesetzten Überschuß an Butandiol-1,4.

Erfindungsgemäß kann nun die unerwünschte Bildung von Tetrahydrofuran auf einen Wert von weniger als 1 % im Reaktionsgemisch reduziert werden, wenn man die Veresterung im Temperaturbereich von 100-130°C, bevorzugt 105-125°C, durchführt. Zulässige Säurezahlen im Veresterungsgemisch, die hierbei erreicht werden, sind 20-50 mg KOH/g. Hierzu werden Butandiol-1,4 und MSA in einem molaren Verhältnis von 1,1-2:1, bevorzugt 1,15-1,5:1 miteinander umgesetzt. Der im obigen Formelschema angegebene Index n nimmt hierbei Werte von 1-10, bevorzugt 2-7, als Durchschnittswerte ein, wobei dieser Durchschnitt eine an sich bekannte Verteilung beinhaltet.

Die Veresterung wird im Temperaturbereich von 100-130°C, bevorzugt 105-125°C, in 1-4 Veresterungsstufen in diskontinuierlicher oder kontinuierlicher Weise durchgeführt. In bevorzugter Weise wird in zwei oder drei Veresterungsstufen und in kontinuierlicher Weise verestert. Für den Fall, daß mehr als eine Veresterungsstufe betrieben wird, kann beispielsweise mit mehreren in Kaskade geschalteten Veresterungsreaktoren gearbeitet werden. Übermäßig lange Reaktionszeiten können dadurch vermieden werden, daß durch schnell laufende Rührsysteme oder durch Anwendung eines Treibgases, beispielsweise Stickstoff, die Austragung des bei der Oligoveresterung entstehenden Reaktionswassers verkürzt wird. Grundsätzlich ist es ebenso möglich, Schleppmittel, beispielsweise Toluol oder ähnliche, dem Fachmann bekannte Schleppmittel, einzusetzen, um in einer azeotropen Destillation das Spaltwasser aus dem Reaktionsgemisch zu entfernen. Es ist jedoch ein Vorteil des erfindungsgemäßen Verfahrens, daß auf solche Schleppmittel verzichtet werden kann.

Die Wirksamkeit der erfindungsgemäßen Veresterung kann in vorteilhafter Weise weiter gesteigert werden, wenn man die Kaskade der Reaktionsapparate mit einer Kaskade der Reaktionstemperatur kombiniert. So hat es sich erfindungsgemäß als vorteilhaft erwiesen, die Veresterungsreaktion zunächst bei einer möglichst niedrigen Reaktionstemperatur im Rahmen des angegebenen Bereichs zu beginnen, um sie in einer weiteren oder in mehreren weiteren Stufen zu erhöhen, beispielsweise von Stufe zu Stufe um 5-30°C, bevorzugt um 5-20°C, besonders bevorzugt um 5-10°C.

Um die Austragung des bei der Veresterung herausgespaltenen Wassers zu beschleunigen, ist es nicht sinnvoll, bei großem Überdruck zu arbeiten; es ist im Gegenteil förderlich, einen Unterdruck anzulegen. Erfindungsgemäß kommt daher der Gesamtbereich von 1500-100 mbar in Frage. Hierbei wird mit Vorteil die erste Veresterungsstufe von mehreren Stufen bei Normaldruck und die letzte Veresterungsstufe bei Unterdruck, beispielsweise bei 200-500 mbar, durchgeführt. Für den Fall, daß mehr als zwei Stufen eingesetzt werden, können die mittleren Veresterungsstufen sowohl bei Normaldruck als auch bei Unterdruck betrieben werden. Eine beispielhafte Kombination von drei Veresterungsstufen sieht Normaldruck für die erste Stufe, 400-1000 mbar für die zweite Veresterungsstufe und 200-500 mbar für die dritte Veresterungsstufe vor.

Die für die Veresterung in Frage kommenden Reaktionsapparate bestehen jeweils aus einem Reaktionsgefäß aus säurefestem Material, das mit einem wirksamen Rührer und einer aufgesetzten Destillationskolonne üblicher Bauart mit 8 bis 15 Böden ausgerüstet ist.

Zum Einsatz im erfindungsgemäßen Verfahren eignet sich sowohl reines MSA mit einer Reinheit von mehr als 99 % als auch ein MSA, das bis zu 15 Gew.-% Maleinsäure enthält. Das eingesetzte Butandiol-1,4 hat mit Vorteil ebenfalls eine Reinheit von mehr als 99 %; jedoch sind auch hier Gehalte an Wasser und/oder Tetrahydrofuran und/oder γ-Butyrolacton zulässig, wie sie etwa in Destillationsrückläufen vorkommen. Auch ein Gehalt an recyclisiertem oligomeren Butandiol-1,4-maleinat ist zulässig. Alle Fremdstoffanteile sind bei der Festlegung der molaren Verhältnisse zu berücksichtigen.

Das wie oben hergestellte oligomere Butandiol-1,4-maleinat ist frei von reaktionsfremden Stoffen und kann daher ohne zusätzliche Reinigungsschritte direkt in die folgende Hydrierung eingespeist werden.

Die Hydrierungsschritte im erfindungsgemäßen Verfahren erfolgen in der flüssigen Phase mit überschüssigem Wasserstoff (20-100facher molarer H₂-Überschuß). Durch die flüssige Phase wird der Energieaufwand für Gasphasenprozesse kostensparend vermieden. Die erfindungsgemäße Hydrierung erfolgt ferner in zwei separaten Schritten mit unterschiedlichen Temperaturbereichen und unterschiedlichen Katalysatoren. Beide Katalysatoren werden in Form von körnigen, trägerfreien Katalysatoren aus verpreßtem Metallpulver bzw. Oxidpulver angewandt. Dies erlaubt die Durchführung in diskontinuierlicher oder kontinuierlicher, bevorzugt in kontinuierlicher Weise, wobei die Schwierigkeiten mit pulverförmigen Katalysatoren entfallen, nämlich die Schwierigkeit, Pulverkatalysatoren gezielt und gleichmäßig zu aktivieren, die Schwierigkeit, Pulverkatalysatoren mit Hilfe von speziellen Schlammpumpen umzuwälzen, und die Schwierigkeit, Pulverkatalysatoren vom Reaktionsprodukt quantitativ abzutrennen. Schlammpumpen unterliegen nämlich einer hohen mechanischen Beanspruchung. Die quantitative Entfernung pulverförmiger Katalysatoren ist weiterhin aufwendig, weil sie eine Grob- und ein Feinfiltration mit Apparaten in Wechselausführung erfordert. Ferner ist die Gefahr groß, daß die Katalysatoren durch diese zusätzlichen Operationen schnell ihre Aktivität verlieren und daher weiterhin hohe Katalysatorverbräuche in Kauf zu nehmen sind. Im Gegensatz zu diesen aufgezeigten Schwierigkeiten besitzen die erfindungsgemäß einzusetzenden Katalysatoren eine hohe Säureunempfindlichkeit, eine hohe Druckunempfindlichkeit und weisen eine hohe Aktivität auf, die auch über einen Zeitraum von 1 bis mehreren Jahren nicht nachläßt. Der letztere Vorteil ist sehr wichtig, da auch bei Festbettreaktoren ein häufiger Katalysatorwechsel sehr aufwendig ist.

Im ersten Hydrierschritt wird im Temperaturbereich von 60-130°C und im zweiten Hydrierschritt bei einer Temperatur von 190-230°C gearbeitet, wobei der Temperaturunterschied zwischen dem ersten und dem zweiten Hydrierschritt 60-130°C beträgt. Der Druckbereich für beide Hydrierschritte ist 100-400 bar, bevorzugt 150-300 bar, wobei jedoch in beiden Hydrierschritten bei gleichem oder bei unterschiedlichem Druck in diesem Bereich gearbeitet werden kann.

Als Katalysatoren für den ersten Hydrierschritt werden trägerfreie Katalysatoren aus verpreßtem Metallpulver von Nickel, Eisen, Kobalt oder Gemischen mehrerer hiervon eingesetzt. Der Begriff Gemisch umfaßt hierbei ein Gemisch von Pulvern der einzelnen Metalle und Pulver von vorher erschmolzenen Zweier- oder Dreier-Legierungen dieser Metalle. In bevorzugter Weise wird ein verpreßtes Metallpulver aus Nickel, aus einem Gemisch von Nickel mit Eisen, Kobalt oder Eisen-Kobalt-Gemisch oder aus einem Kobalt-Eisen-Gemisch eingesetzt. Für den Fall des Einsatzes einer Nickellegierung oder einem Gemisch von Nickelpulver mit Pulvern der anderen genannten Metalle, liegt der Nickelanteil bei 60-90 Gew.-% der gesamten Legierung. Für den Fall einer Kobalt-Eisen-Legierung oder einem Gemisch dieser Metallpulver liegt der Kobaltgehalt bei 60-90 Gew.-% der gesamten Legierung/des gesamten Gemisches. Reine Metalle oder Legierungen aus reinen Metallen sind jedoch recht teuer, so daß nach kostengünstigeren Alternativen gesucht wurde. Dabei wurde gefunden, daß die zum Einsatz kommenden Pulver der Metalle oder ihrer Legierungen zusätzlich Anteile anderer, nicht katalytisch wirkender Metalle enthalten können, ohne daß die hohe Aktivität gemindert wird. Die Summe der nicht katalytisch wirkenden Anteile, beispielsweise Si, Al und Ti darf bis zu 10 Gew.-% der Summe der katalytisch wirksamen Metalle betragen.

Im zweiten Hydrierschritt werden als fest angeordnete Katalysatoren trägerfreie Formkörper aus verpreßten Pulvern von Kupferoxiden, Zinkoxid und Aluminiumoxid mit Anteilen von Oxiden des Nickels, Eisens und Kobalts oder Gemischen mehrerer von ihnen eingesetzt. Hierbei beträgt der Kupferanteil (gerechnet als Metall) 40-60 Gew.-%, der Zinkanteil 15-30 Gew.-% und der Aluminiumanteil 0,2-6 Gew.-%, bezogen auf die Gesamtmenge des verpreßten Pulvers. Der Gehalt an Nickel, Eisen, Kobalt oder einem Gemisch mehrerer von ihnen beträgt 0,1-1 Gew.-%, bevorzugt 0,2-0,5 Gew.-%, alles gerechnet als Metall. Alkali-und Erdalkalimetalle dürfen bis zu einem Anteil von 0,1 Gew.-% anwesend sein. Der Rest zu 100 Gew.-% ist Sauerstoff für die in oxidischer Form vorliegenden Metalle.

Die Herstellung der trägerfreien Formkörper kann nach gebräuchlichen Methoden durch Verpressen der Metallpulver bzw. Metalloxidpulver, beispielsweise auf Tablettier- oder Pelletier-Maschinen, unter hohem Druck erfolgen, wobei zur Verbesserung des Haftvermögens der Metallpartikel bzw. Metalloxidpartikel auch Graphit und/oder Klebstoffe in Mengen von 0,5-1 Gew.-%, bezogen auf das Gesamtgewicht der zu verpressenden Bestandteile, zum Einsatz kommen können. Die Herstellung und Aufbewahrung der trägerfreien Formkörper aus verpreßten Metallpulvern erfolgt vorzugsweise in einer sauerstofffreien Atmosphäre, um Oberflächenoxidation zu vermeiden. Beispiele für Formkörper für beide Hydrierschritte sind Tabletten, Kugeln oder Granulate mit Durchmessern von 3-7 mm. Tablettierte Formkörper können weiterhin zur Vergrößerung der äußeren Oberfläche mit einer axialen Durchbohrung versehen sein Solche Formkörper haben makroskopisch betrachtet eine glatte Oberfläche. Die in beiden Hydrierschritten eingesetzten verpreßten Formkörper haben eine hohe Druckfestigkeit auf die Formkörperoberfläche. Die im ersten Hydrierschritt eingesetzten verpreßten Metallpulver haben eine Druckfestigkeit von 50-500 N, bevorzugt von 100-400 N auf die gewölbte Formkörperoberfläche. Die im zweiten Reaktor eingesetzten verpreßten Metalloxidpulver haben eine Druckfestigkeit von 50-200 N, bevorzugt 75-150 N auf die gewölbte Formkörperoberfläche. Die innere Oberfläche der verpreßten Metallpulver für den ersten Hydrierschritt beträgt 10-90 m²/g. Die innere Oberfläche der im zweiten Hydrierschritt eingesetzten verpreßten Metalloxidpulver beträgt 30-80 m²/g. Die Druckfestigkeit der trägerfreien Formkörper kann nach DIN 50 106 bestimmt werden. Die Bestimmung der inneren Oberflächen erfolgt nach F.M. Nelsen und F.T. Eggertsen, Analyt. Chem. 30 (1958), 1387 bzw. S.J. Gregg und S.W. Sing, Adsorption, Surface Area and Porosity, London 1967, Kap. 2 und 8.

Während es prinzipiell gleich ist, das zu hydrierende Butandiol-1,4-maleinat in den aufrecht stehenden Hydrierreaktoren von unten nach oben oder von oben nach unten und in den beiden Hydrierreaktoren unabhängig voneinander strömen zu lassen, hat es sich als vorteilhaft herausgestellt, das zu hydrierende oligomere Butandiol-1,4-maleinat im ersten Reaktor von unten nach oben aufsteigend zu bewegen und im zweiten Reaktor von oben nach unten absteigend, d.h. in der Rieselphase. Dabei kann man das zu hydrierende Butandiol-1,4-maleinat entweder gemeinsam mit dem vorher zugemischten Wasserstoff über den Katalysator strömen lassen (Gleichstromverfahren) oder das Butandiol-1,4-maleinat dem Wasserstoff entgegenführen (Gegenstromverfahren).

Die Hydrierreaktoren können einzelne Hochdruckrohre aus Stahl oder einer Stahllegierung sein, die mit den Formkörpern ganz oder teilweise gefüllt werden, wobei bei größeren Rohrquerschnitten auch die Anwendung der trägerfreien Formkörper auf Horden (etwa Drahtkörbe oder ähnliches) nützlich sein kann; man kann jedoch auch Hochdruckrohrbündel innerhalb eines gemeinsamen Mantels anwenden, wobei die Einzelrohre wiederum mit den trägerfreien Formkörpern ganz oder teilweise gefüllt werden.

Während die in der ersten Hydrierstufe als Katalysatoren eingesetzten Formkörper aus verpreßten Metallpulvern ohne weitere Aktivierung zum Einsatz kommen, müssen die im zweiten Reaktor als Katalysatoren eingesetzten Formkörper aus verpreßten Oxidpulvern vor ihrem Einsatz sorgfältig reduziert werden. Dies geschieht durch den Einsatz eines Reduktionsgases, das aus einer Inertgas/Wasserstoff-Mischung besteht, in der der Wasserstoffgehalt zu Beginn bei 10-15 Vol.-% liegt. Als Inertgas wird bevorzugt Stickstoff eingesetzt. Die Reduktion erfolgt beispielsweise innerhalb eines Zeitraums von etwa 24 Stunden bei einer Reduktionstemperatur von 180-200°C, wobei in der Endphase der Reduktion der Stickstoffanteil der Gasmischung mehr und mehr vermindert wird, bis schließlich reiner Wasserstoff den Reaktor zur Reduktion durchströmt. Die Reduktion ist beendet, wenn der Katalysator keinen Wasserstoff mehr verbraucht und infolgedessen kein Reaktionswasser mehr bildet.

Die stündliche Hydrierkatalysatorbelastung kann in beiden Hydrierstufen bei 200-400 g Butandiol-1,4-maleinat pro Liter Katalysator liegen. Im Rahmen des erfindungsgemäßen Verfahrens sind hohe Katalysatorstandzeiten von 12 000-16 000 Stunden zu erreichen.

Das die zweite Hydrierstufe verlassende Reaktionsgemisch besteht nach der Entspannung, bei welcher man den überschüssigen Wasserstoff abfangen und nach erfolgter Kompression und Ergänzung des verbrauchten Wasserstoffs erneut zum Einsatz bringen kann, zu 97 Gew.-% und mehr aus Butandiol-1,4. Es enthält an organischen Leichtsiedern neben maximal 0,5 Gew.-% Tetrahydrofuran, maximal 0,1 Gew.-% n-Butanol und maximal 0,4 Gew.-% γ-Butyrolacton sowie untergeordnete Mengen an Höhersiedern von maximal 1,0 Gew.-% eines höhermolekularen Rückstandes sowie maximal 1,0 Gew.-% H₂O. γ-Butyrolacton und Höhersieder werden wieder in den Prozeß zurückgeführt, so daß dessen Gesamtselektivität bezüglich Butandiol-1,4 bei mehr als 98 Gew.-% liegt.

Das erzeugte Butandiol-1,4 wird nach der destillativen Entfernung von Leicht-und Hochsiedern in einer Reinheit von mehr als 99,8 Gew.-% erhalten und ist in dieser Reinheit für alle weiterverarbeitenden chemischen Prozesse einsetzbar.

### Beispiele

### Beispiel 1

In den ersten Reaktionsapparat einer Reaktionsapparatekaskade aus säurefestem Material, die aus 3 hintereinandergeschalteten Reaktionsapparaten mit einem Volumen von jeweils 5 l bestand, die jeweils mit einem Schnellrührsystem üblicher Bauart (Turbinenrührer, Drehzahl: 800-1 200/min) und einer Destillationskolonne (10 theoret. Böden) versehen waren, wurde eine Lösung von 768 g (7,83 Mol) Maleinsäureanhydrid (MSA) in 1 059 g (11,75 Mol) Butandiol-1,4 gegeben, unter Rühren schnell auf eine Reaktionstemperatur von 105-100°C erhitzt und das sich bildende Reaktionswasser abdestilliert. Nach 6 Stunden Reaktionszeit wurde das Reaktionsgemisch in den angeschlossenen zweiten Reaktionsapparat gebracht; dort wurde bei einer Reaktionstemperatur von 110-115°C unter Rühren ein weiterer Teil des Reaktionswassers abdestilliert. Nach 4 Stunden Reaktionszeit wurde das Reaktionsgemisch schließlich in den angeschlossenen dritten Reaktionsapparat gebracht; dort wurde unter einem Druck von 400 mbar und bei einer Reaktionstemperatur, die allmählich von 115 auf 125°C gesteigert wurde, der Rest des Reaktionswassers entfernt. Nach weiteren 2 Stunden (insgesamt 12 Stunden) wurde die Veresterung beendet. Das erhaltene oligomere Butandiol-1,4-maleinat (1 682 g) hatte einen mittleren Oligomerisierungsgrad von n = 3 (gemessen durch Gelpermeabilitätschromatographie) und eine Säurezahl von 21 mg KOH/g Reaktionsgemisch.

Unmittelbar nach dem Entleeren des ersten Reaktionsapparates kann der Veresterungsprozeß erneut gestartet und auf die gleiche Weise kontinuierlich fortgesetzt werden.

### Beispiel 2

In den ersten Reaktionsapparat der gleichen Reaktionsapparatekaskade wie im Beispiel 1 wurde eine Lösung von 899 g (9,17 Mol) MSA in 950 g (10,5 Mol) Butandiol-1,4 gegeben, unter Rühren auf eine Reaktionstemperatur von 105-110°C erhitzt und das sich bildende Reaktionswasser abdestilliert. Nach 8 Stunden Reaktionszeit wurde das Reaktionsgemisch in den angeschlossenen zweiten Reaktionsapparat abgelassen; dort wurde bei einer Reaktionstemperatur von 110-115°C ein weiterer Teil des Reaktionswassers abdestilliert. Nach 2 Stunden Reaktionszeit wurde das Reaktionsgemisch schließlich in den dritten Reaktionsapparat abgelassen; dort wurde unter einem Druck von 350 mbar und bei einer Reaktionstemperatur von 115-120°C der Rest des Reaktionswassers entfernt. Nach einer Reaktionszeit von weiteren 2 Stunden (insgesamt 12 Stunden) war die Veresterung beendet. Das erhaltene oligomere Butandiol-1,4-maleinat (1 684 g) hatte einen mittleren Oligomerisierungsgrad von n = 5 (Molmassenverteilung gemessen durch Gelpermeabilitätschromatographie) und eine Säurezahl von 42 mg KOH/g Reaktionsgemisch.

### Beispiel 3

Ein senkrecht stehendes, wärmeisoliertes Hochdruckrohr aus nichtrostendem, säurefestem Stahl von 45 mm Innendurchmesser und 1 m Länge, das vorher mit Stickstoff sauerstofffrei gespült worden war, wurde mit 1,4 l eines durch Tablettierung einer pulverisierten Nickel-Eisenlegierung hergestelltem Hydrierungskatalysator gefüllt. Die Legierung enthielt einen Eisenanteil in Nickel von 15 Gew.-%. Die Tabletten hatten bei einer Zylinderhöhe von 5 mm und einem Durchmesser von 5 mm eine Druckfestigkeit von 132 N auf die Zylindermantelfläche und eine innere Oberfläche von 81 m²/g.

Diesem ersten Hochdruckrohr wurde über eine feste Hochdruckleitung, die über einen elektrisch beheizbaren Wärmeaustauscher geführt wurde, ein zweites senkrecht stehendes, warmeisoliertes Hochdruckrohr aus nichtrostendem, säurefestem Stahl von 45 mm Innendurchmesser und 1 m Länge nachgeschaltet, das mit 1,4 l eines durch Tablettierung von Pulvern von Kupfer-, Zink-, Aluminium- und Eisenoxiden hergestellten Hydrierungskatalysators gefüllt worden war. Der Kupfergehalt der Tabletten lag bei 43 Gew.-%, der Zinkgehalt bei 16 Gew.-%, der Aluminiumgehalt bei 2,2 Gew.-% und der Eisengehalt bei 0,25 Gew.-% (Rest Sauerstoff). Die Tabletten hatten bei einer Zylinderhöhe von 5 mm und einem Durchmesser von 5 mm eine Druckfestigkeit von 120 N auf die Zylindermantelfläche und eine innere Oberfläche von 72 m²/g.

Zur Aktivierung des eine Mischung von Metalloxiden enthaltenden Katalysators wurden die Tabletten zunächst 6 Stunden im Stickstoffstrom getrocknet (Temperatur: max. 200°C, Menge: 5 Nm³N₂/h). Die eigentliche Aktivierung erfolgte unter einem Stickstoffdruck von 200 bar bei einer Temperatur zwischen 180 und 200°C, wobei dem Inertgas allmählich Wasserstoff zugemischt wurde, dessen Zumischungsanteil in der Anfangsphase 10-15 Vol.-% nicht übersteigen durfte. Innerhalb von 24 Stunden wurde der Stickstoffanteil der Gasmischung mehr und mehr vermindert, bis schließlich reiner Wasserstoff den Reaktor durchströmte. Die Reaktion war beendet, wenn kein Reaktionswasser, das in einem nachgeschalteten Abscheider gesammelt wurde, mehr gebildet wurde.

Nach der Aktivierung des eine Mischung von Metalloxiden enthaltenden Hydrierkatalysators wurde das im ersten Hydrierreaktor befindliche Inertgas Stickstoff gegen reinen Wasserstoff ausgetauscht und der Wasserstoffdruck in den beiden Reaktorsystemen, die so miteinander verknüpft waren, das das zu hydrierende Butandiol-1,4-maleinat das erste Hochdruckrohr von unten nach oben aufsteigend passieren mußte und das zweite Hochdruckrohr von oben nach unten absteigend, auf 250 bar erhöht.

Anschließend wurden stündlich 420 g Butandiol-1,4-maleinat, das nach Beispiel 1 gewonnen worden war, gemeinsam mit 5 Nm³ Wasserstoff unter einem Druck von 250 bar durch die hintereinandergeschalteten Hochdruckrohre gepumpt, wobei das Butandiol-1,4-maleinat vor Eintritt in das erste Hochdruckrohr in einem vorgeschalteten elektrisch beheizten Wärmeaustauscher auf eine Temperatur von 110°C erhitzt wurde; das das erste Hochdruckrohr verlassende Reaktionsprodukt wurde vor Eintritt in das zweite Hochdruckrohr auf 210°C erhitzt.

Das das zweite Reaktionsrohr verlassende Reaktionsprodukt (Roh-Butandiol-1,4) wurde in einem dritten Wärmeaustauscher (Wasserkühler) unter 250 bar Wasserstoffdruck auf eine Temperatur <60°C abgekühlt und in einem Gasabscheider vom überschüssigen Wasserstoff, der wieder in das Hydriersystem zurückgeführt wurde, getrennt.

Nach weiterer Abkühlung auf eine Temperatur <30°C und Entspannung auf Normaldruck wurde das Reaktionsprodukt gaschromatographisch untersucht.

Es enthielt an organischen Leichtsiedern 0,4 Gew.-% Tetrahydrofuran, 0,05 Gew.-% n-Butanol und 0,3 Gew.-% γ-Butyrolacton sowie an Höhersiedern 0,4 Gew.-% nicht umgesetztes Butandiol-1,4-maleinat, so daß der Butandiol-1,4-Gehalt des organischen Reaktionsproduktes bei 98,85 Gew.-% lag.

Das erzeugte Butandiol-1,4 wurde nach der destillativen Entfernung von Leicht-und Hochsiedern in einer Reinheit von 99,9 Gew.-% erhalten.

Da das γ-Butyrolacton sowie das nicht umgesetzte Butandiol-1,4-maleinat wieder in den Prozeß zurückgeführt werden konnten, lag dessen Gesamtselektivität bezüglich Butandiol-1,4 bei 99,55 Gew.-%.

Die Katalysatoren waren nach einer Laufzeit von 4 600 Stunden unverändert wirksam, so daß sich die Zusammensetzung des Reaktionsproduktes über diesen Zeitraum nicht veränderte.

### Beispiel 4

Ein Hochdruckrohr wie in Beispiel 3 wurde unter Inertgas mit 1,4 l eines durch Tablettierung von Nickelpulver, das zusätzlich einen Aluminiumgehalt von 5,8 Gew.-% als Binder aufwies, hergestellten Hydrierungskatalysators gefüllt. Die Tabletten hatten bei einer Zylinderhöhe von 3 mm und einem Durchmesser von 3 mm eine Druckfestigkeit von 147 N auf die Zylindermantelfläche und eine innere Oberfläche von 33 m²/g.

Dem ersten Hochdruckrohr wurde ein zweites Hochdruckrohr wie in Beispiel 3 nachgeschaltet, das mit 1,4 l eines durch Tablettierung von Pulvern von Kupfer-, Zink-, Aluminium- und Nickeloxiden hergestellten Hydrierungskatalysators gefüllt worden war. Der Kupfergehalt der Tabletten lag bei 51 Gew.-%, der Zinkgehalt bei 27 Gew.-%, der Aluminiumgehalt bei 0,5 Gew.-% und der Nickelgehalt bei 0,25 Gew.-% (Rest Sauerstoff). Die Tabletten hatten bei einer Zylinderhöhe von 3 mm und einem Durchmesser von 3 mm eine Druckfestigkeit von 75 N auf die Zylindermantelfläche und eine innere Oberfläche von 35 m²/g.

Nach der Aktivierung des eine Mischung von Metalloxiden enthaltenden Hydrierkatalysators wie in Beispiel 3 wurde das im ersten Hydrierreaktor befindliche Inertgas Stickstoff gegen reinen Wasserstoff ausgetauscht und der Wasserstoffdruck in beiden Reaktionssystemen, die in der gleichen Weise wie in Beispiel 3 miteinander verknüpft waren, auf 300 bar erhöht.

Anschließend wurden stündlich 560 g Butandiol-1,4-maleinat, das nach Beispiel 2 gewonnen worden war, gemeinsam mit 5 Nm³ Wasserstoff unter einem Druck von 300 bar kontinuierlich durch die hintereinandergeschalteten Hochdruckrohre gepumpt, wobei das Butandiol-1,4-maleinat vor Eintritt in das erste Hochdruckrohr auf eine Temperatur von 100°C erhitzt wurde und das das erste Hochdruckrohr verlassende Reaktionsprodukt vor Eintritt in das zweite Hochdruckrohr auf 200°C.

Das das zweite Reaktionsrohr verlassende Reaktionsprodukt (Roh-Butandiol-1,4) enthielt nach der Abtrennung von überschüssigem Wasserstoff und der Abkühlung auf eine Temperatur <30°C laut gaschromatographischer Analyse an organischen Leichtsiedern 0,45 Gew.-% Tetrahydrofuran, 0,07 Gew.-% n-Butandiol und 0,4 Gew.-% γ-Butyrolacton sowie an Höhersiedern 0,35 Gew.-% nicht umgesetztes Butandiol-1,4-maleinat, so daß der Butandiol-1,4-Gehalt des organischen Reaktionsproduktes bei 98,73 Gew.-% lag.

Nach der destillativen Entfernung von Leicht- und Hochsiedern wurde das erzeugte Butandiol-1,4 in einer Reinheit von 99,9 Gew.-% erhalten.

Da das γ-Butyrolacton sowie das nicht umgesetzte Butandiol-1,4-maleinat wieder in den Prozeß zurückgeführt werden konnten, lag dessen Gesamtselektivität bezüglich Butandiol-1,4 bei 99,48 Gew.-%.

Die Katalysatoren waren nach einer Laufzeit von 5 800 Stunden unverändert wirksam, so daß sich die Zusammensetzung des Reaktionsproduktes über diesen Zeitraum nicht veränderte.

### Beispiel 5

Ein Hochdruckrohr wie in Beispiel 3 wurde unter Inertgas mit 1,4 l eines durch Tablettierung einer pulverisierten Nickel-Kobalt-Legierung hergestellten Hydrierungskatalysators gefüllt. Die Legierung enthielt einen Kobaltanteil in Nickel von 10 Gew.-%. Die Tabletten hatten bei einer Zylinderhöhe von 5 mm und einem Durchmesser von 5 mm eine Druckfestigkeit von 187 N auf die Zylindermantelfläche und eine innere Oberfläche von 68 m²/g.

Dem ersten Hochdruckrohr wurde ein zweites Hochdruckrohr wie in Beispiel 3 nachgeschaltet, das mit 1,4 l eines durch Tablettierung von Pulvern von Kupfer-, Zink-, Aluminium- und Kobaltoxiden hergestellten Hydrierungskatalysators gefüllt worden war. Der Kupfergehalt der Tabletten lag bei 49 Gew.-%, der Zinkgehalt bei 29 Gew.-%, der Aluminiumgehalt von 1,3 Gew.-% und der Kobaltgehalt bei 0,22 Gew.-%. Die Tabletten hatten bei einer Zylinderhöhe von 5 mm und einem Durchmesser von 5 mm eine Druckfestigkeit von 108 N auf die Zylindermantelfläche und eine innere Oberfläche von 49 m²/g.

Nach der Aktivierung des oxidischen Hydrierkatalysators wie im Beispiel 3 wurde das im ersten Hydrierreaktor befindliche Inertgas Stickstoff gegen reinen Wasserstoff ausgetauscht und der Wasserstoffdruck in beiden Reaktionssystemen, die in der gleichen Weise wie im Beispiel 3 miteinander verknüpft waren, auf 300 bar erhöht.

Anschließend wurden stündlich 420 g Butandiol-1,4-maleinat, das nach Beispiel 1 gewonnen worden war, gemeinsam mit 5 Nm³ Wasserstoff unter einem Druck von 300 bar kontinuierlich durch die hintereinandergeschalteten Hochdruckrohre gepumpt, wobei das Butandiol-1,4-maleinat vor Eintritt in das erste Hochdruckrohr auf eine Temperatur von 120°C erhitzt wurde und das das erste Hochdruckrohr verlassende Reaktionsprodukt vor Eintritt in das zweite Hochdruckrohr auf 220°C.

Das das zweite Reaktionsrohr verlassende Reaktionsprodukt (Roh-Butandiol-1,4) enthielt nach der Abtrennung von überschüssigem Wasserstoff und der Abkühlung auf eine Temperatur <30°C laut gaschromatographischer Analyse an organischen Leichtsiedern 0,48 Gew.-% Tetrahydrofuran, 0,09 Gew.-% n-Butanol und 0,45 Gew.-% γ-Butyrolacton sowie an Höhersiedern 0,48 Gew.-% nicht umgesetztes Butandiol-1,4-maleinat, so daß der Butandiol-1,4-Gehalt des organischen Reaktionsproduktes bei 98,50 Gew.-% lag.

Da das γ-Butyrolacton sowie das nicht umgesetzte Butandiol-1,4-maleinat wieder in den Prozeß zurückgeführt werden konnten, lag dessen Gesamtselektivität bezüglich Butandiol-1,4 bei 99,43 Gew.-%.

Die Katalysatoren waren nach einer Laufzeit von 3 600 Stunden unverändert wirksam, so daß sich die Zusammensetzung des Reaktionsproduktes über diesen Zeitraum nicht veränderte.

## Patentansprüche

1. Verfahren zur Herstellung von Butandiol-1,4 aus Maleinsäureanhydrid (MSA) durch Veresterung des MSA und katalytische Hydrierung des entstandenen Esters in der Flüssigphase, dadurch gekennzeichnet, daß man
a) zur Veresterung des MSA Butandiol-1,4 im molaren Verhältnis Butandiol-1,4:MSA = 1,1-2:1, bevorzugt 1,15-1,5:1, einsetzt,
b) die Veresterung unter Abdestillieren des herausgespaltenen Wassers diskontinuierlich oder kontinuierlich in 1-4 Veresterungsstufen im Temperaturbereich von 100-130°C, bervorzugt 105-125°C, und im Druckbereich von 1500-100 mbar durchführt,
c) den im Schritt b) gebildeten Oligoester in einem ersten Hydrierschritt im Temperaturbereich von 60-130°C an einem trägeifreien Katalysator aus verpreßtem Metallpulver von Ni, Fe, Co oder Gemischen daraus mit überschüssigem Wasserstoff behandelt und
d) in einem zweiten Hydrierschritt im Temperaturbereich von 190-230°C an einem reduzierten, trägerfreien Katalysator aus verpreßten Pulvern von Cu-Oxiden, ZnO und Al₂O₃ mit Anteilen von Oxiden des Ni, Fe, Co oder Gemischen mehrerer von ihnen die Hydrierung vervollständigt, wobei der Temperaturunterschied zwischen den Schritten c) und d) 60-130°C beträgt, ein 20-100facher molarer H₂-Überschuß angewandt wird und die Schritte c) und d) bei gleichem oder unterschiedlichem Druck im Bereich von 100-400 bar, bevorzugt 150-300 bar, durchgeführt werden.

2. Verfahren nach Anspruch 1, dadurch gekennzeichnet, daß bei mehrstufiger Veresterung die Temperatur von Stufe zu Stufe um 5-30°C, bevorzugt um 5-20°C, besonders bevorzugt um 5-10°C, ansteigt.

3. Verfahren nach Anspruch 1, dadurch gekennzeichnet, daß in zwei oder drei Veresterungsstufen kontinuierlich gearbeitet wird.

4. Verfahren nach Anspruch 3, dadurch gekennzeichnet, daß die erste Veresterungsstufe bei Normaldruck, die letzte Veresterungsstufe bei 200-500 mbar und im Falle von drei Veresterungsstufen die mittlere Veresterungsstufe bei 400-1000 mbar durchgeführt wird.

5. Verfahren nach Anspruch 1, dadurch gekennzeichnet, daß als Katalysator für die erste Hydrierstufe ein verpreßtes Metallpulver aus Ni, aus einem Gemisch von Ni mit Fe, Co oder Fe-Co-Gemisch mit einem Ni-Gehalt von 60-90 Gew.-% der gesamten Legierung oder aus einer Co-Fe-Legierung mit einem Co-Gehalt von 60-90 Gew.-% der gesamten Legierung eingesetzt wird.

6. Verfahren nach Anspruch 1, dadurch gekennzeichnet, daß der im ersten Hydrierschritt eingesetzte Katalysator aus verpreßtem Metallpulver eine Druckfestigkeit von 50-500 N, bevorzugt 100-400 N, auf die gewölbte Formkörperoberfläche und eine innere Oberfläche von 10-90 m²/g aufweist.

7. Verfahren nach Anspruch 1, dadurch gekennzeichnet, daß als Katalysator für die zweite Hydrierstufe ein verpreßtes Gemisch aus Metalloxidpulvern eingesetzt wird, dessen Cu-Anteil 40-60 Gew.-%, dessen Zn-Anteil 15-30 Gew.-%, dessen Al-Anteil 0,2-6 Gew.-% und dessen Anteil an Ni, Fe, Co oder einem Gemisch mehrerer von ihnen 0,1-1 Gew.-%, bevorzugt 0,2-0,5 Gew.-%, betragen, wobei alle Angaben auf die Gesamtmenge des Oxidpulvers bezogen sind und der Rest zu 100 Gew.-% Sauerstoff darstellt.

8. Verfahren nach Anspruch 1, dadurch gekennzeichnet, daß der im zweiten Hydrierschritt eingesetzte Katalysator aus verpreßten Metalloxidpulvern eine Druckfestigkeit von 50-200 N, bevorzugt 75-150 N, auf die gewölbte Formkörperoberfläche und eine innere Oberfläche von 30-80 m²/g aufweist.

9. Verfahren nach Anspruch 1, dadurch gekennzeichnet, daß man den im Schritt b) gebildeten Oligoester ohne Reinigung zur Hydrierung einsetzt.

10. Verfahren nach Anspruch 1, dadurch gekennzeichnet, daß der Katalysator für den zweiten Hydrierschritt d) vor seinem Einsatz durch eine Inertgas/Wasserstoff-Mischung bei 180-200°C reduziert wird.

## Claims

1. Process for preparing 1,4-butanediol from maleic anhydride (MA) by esterification of the MA and catalytic hydrogenation of the ester formed in the liquid phase, characterized in that
a) the MA is esterified using 1,4-butanediol in a molar ratio of 1,4-butanediol:MA = 1.1-2:1, preferably 1.15-1.5:1,
b) the esterification is carried out while distilling off the eliminated water, batchwise or continuously in 1-4 esterification stages in the temperature range of 100-130°C, preferably 105-125°C and in the pressure range of 1500-100 mbar,
c) the oligoester formed in step b) is treated with excess hydrogen in a first hydrogenation step in the temperature range of 60-130°C over a support-free catalyst of compacted metal powder of Ni, Fe, Co or mixtures thereof, and
d) the hydrogenation is completed in a second hydrogenation step in the temperature range of 190-230°C over a reduced, support-free catalyst of compacted powders of copper oxides, ZnO and Al₂O₃ containing proportions of oxides of Ni, Fe, Co or mixtures of a plurality of these, with the temperature difference between the steps c) and d) being 60-130°C, a 20 to 100-fold molar H₂ excess being used and the steps c) and d) being carried out at the same or different pressure in the range of 100-400 bar, preferably 150-300 bar.

2. Process according to Claim 1, characterized in that in multistage esterification the temperature rises from stage to stage by 5-30°C, preferably by 5-20°C, particularly preferably by 5-10°C.

3. Process according to Claim 1, characterized in that the esterification is carried out continuously in two or three esterification stages.

4. Process according to Claim 3, characterized in that the first esterification stage is carried out at atmospheric pressure, the last esterification stage is carried out at 200-500 mbar and, in the case of three esterification stages, the middle esterification stage is carried out at 400-1000 mbar.

5. Process according to Claim 1, characterized in that the catalyst used for the first hydrogenation stage is a compacted metal powder of Ni, of a mixture of Ni with Fe, Co or Fe-Co mixture having an Ni content of 60-90% by weight of the total alloy or of a Co-Fe alloy having a Co content of 60-90% by weight of the total alloy.

6. Process according to Claim 1, characterized in that the catalyst of compacted metal powder used in the first hydrogenation step has a compressive strength of 50-500 N, preferably 100-400 N, on the curved surface of the shaped body and an internal surface area of 10-90 m²/g.

7. Process according to Claim 1, characterized in that the catalyst used for the second hydrogenation stage is a compacted mixture of metal oxide powders whose Cu content is 40-60% by weight, whose Zn content is 15-30% by weight, whose Al content is 0.2-6% by weight and whose content of Ni, Fe, Co or a mixture of a plurality thereof is 0.1-1% by weight, preferably 0.2-0.5% by weight, with all percentages being based on the total amount of oxide powder and the remainder to 100% by weight being oxygen.

8. Process according to Claim 1, characterized in that the catalyst of compacted metal oxide powders used in the second hydrogenation step has a compressive strength of 50-200 N, preferably 75-150 N, on the curved surface of the shaped body and an internal surface area of 30-80 m²/g.

9. Process according to Claim 1, characterized in that the oligoester formed in step b) is used without purification for the hydrogenation.

10. Process according to Claim 1, characterized in that the catalyst for the second hydrogenation step d) is reduced at 180-200°C by an inert gas/hydrogen mixture prior to its use.

## Revendications

1. Procédé pour la fabrication de 1,4-butanediol à partir d'anhydride de l'acide maléique (AAM) par estérification de l'AAM et hydrogénation catalytique en phase liquide de l'ester obtenu, caractérisé en ce que:
a) pour l'estérification de l'AAM, on utilise du 1,4-butanediol dans un rapport molaire 1,4-butanediol:MSA = 1,1-2:1, et de préférence 1,15-1,5:1,
b) l'estérification s'effectue en continu ou en discontinu avec séparation par distillation de l'eau qui se dégage, en 1-4 étapes d'estérification, dans la plage des températures de 100-130° C, de préférence de 105-125° C, et dans la plage des pressions de 1500-100 mbar,
c) dans une première étape d'hydrogénation dans la plage des températures de 60-130° C, sur un catalyseur sans support, constitué de poudre métallique précomprimée de Ni, Fe, Co ou de mélanges de ces derniers, l'oligoester formé dans l'étape b) est traité par un excès d'hydrogène, et
d) dans une deuxième étape d'hydrogénation dans la plage des températures de 190-230° C, sur un catalyseur réduit, sans support, constitué d'une poudre comprimée d'oxydes de cuivre, de ZnO et d'Al₂O₃, comportant des teneurs en oxydes de Ni, Fe, Co, ou de mélanges de plusieurs de ceux-ci, l'hydrogénation se termine, tandis que la différence de température entre les étapes c) et d) vaut 60-130° C, qu'on utilise un excès molaire en H₂ de 20-100 fois, et que les étapes c) et d) sont conduites à la même pression ou à des pressions différentes dans la plage de 100-400 bars, et de préférence de 150-300 bars.

2. Procédé selon la revendication 1, caractérisé en ce que dans le cas d'une estérification en plusieurs étapes, la température augmente de 5-30° C d'une étape à la suivante, de préférence de 5-20° C, et de manière particulièrement préférable de 5-10° C.

3. Procédé selon la revendication 1, caractérisé en ce que l'on travaille en continu et en deux ou trois étapes d'estérification.

4. Procédé selon la revendication 3, caractérisé en ce que la première étape d'estérification est conduite sous pression normale, la dernière étape d'estérification à 200-500 mbars, et dans le cas étapes d'estérification, l'étape centrale d'estérification est conduite à 400-1000 mbars.

5. Procédé selon la revendication 1, caractérisé en ce que l'on utilise comme catalyseur pour la première étape d'hydrogénation une poudre métallique comprimée constituée de Ni, d'un mélange de Ni et de Fe, Co ou un mélange de Fe-Co, avec une teneur en Ni de 60-90% en poids pour l'ensemble de l'alliage ou en un alliage de Co-Fe présentant une teneur en Co de 60-90% en poids de l'ensemble de l'alliage.

6. Procédé selon la revendication 1, caractérisé en ce que le catalyseur en poudres métalliques comprimées utilisé dans la première étape d'hydrogénation présente une résistance à la compression de 50-500 N, de préférence de 100-400 N, sur la surface courbe du corps façonné, et une surface interne de 10-90 m2/g.

7. Procédé selon la revendication 1, caractérisé en ce que l'on utilise comme catalyseur pour la deuxième étape d'hydrogénation un mélange comprimé de poudres d'oxydes métalliques dont la teneur en cuivre est de 40-60% en poids, la teneur en zinc de 15-30% en poids, la teneur en Al de 0,2-6% en poids et la teneur en Ni, Fe, Co ou un mélange de plusieurs de ceux-ci de 0,1-1% en poids, de préférence de 0,2-0,5% en poids, tous les pourcentages étant calculés par rapport à la quantité totale de la poudre d'oxydes et le complément pour former 100% en poids étant de l'oxygène.

8. Procédé selon la revendication 1, caractérisé en ce que le catalyseur en poudres d'oxydes métalliques comprimées utilisé dans la deuxième étape d'hydrogénation présente une résistance à la compression de 50-200 N, de préférence de 75-150 N, sur la surface courbe du corps façonné, et une surface interne de 30-80 m²/g.

9. Procédé selon la revendication 1, caractérisé en ce que l'on utilise l'oligo-ester formé au cours de l'étape b) pour l'hydrogénation sans le purifier.

10. Procédé selon la revendication 1, caractérisé en ce que le catalyseur de la deuxième étape d'hydrogénation d) est réduit avant son utilisation par un mélange de gaz inerte et d'hydrogène à 180-200° C.
